Europäisches Patentamt

European Patent Office

Office européen des brevets

(19)

(11) Veröffentlichungsnummer: **0 182 064**
**A1**

# (12) EUROPÄISCHE PATENTANMELDUNG

(21) Anmeldenummer: **85112733.2**

(22) Anmeldetag: **08.10.85**

(51) Int. Cl.⁴: **G 01 N 27/16**, G 21 D 3/04,
G 01 N 33/00

(30) Priorität: **22.10.84 DE 3438659**

(43) Veröffentlichungstag der Anmeldung: **28.05.86**
**Patentblatt 86/22**

(84) Benannte Vertragsstaaten: **CH DE FR LI SE**

(71) Anmelder: **KRAFTWERK UNION**
**AKTIENGESELLSCHAFT, Wiesenstrasse 35,**
**D-4330 Mülheim (Ruhr) (DE)**

(72) Erfinder: **Heck, Reinhard, Dipl.-Ing.,**
**Kurfürstenstrasse 43, D-6450 Hanau (DE)**
Erfinder: **von Gunten, Anton, Dr. Dipl.-Phys.,**
**Mauerstrasse 32, D-6100 Darmstadt (DE)**
Erfinder: **Melchior, Klaus, Dr. Dipl.-Ing.,**
**Dortelweilerstrasse 58, D-6000 Frankfurt/Main 60 (DE)**

(74) Vertreter: **Mehl, Ernst, Dipl.-Ing. et al, Postfach 22 01 76,**
**D-8000 München 22 (DE)**

(54) **Sonde mit einem Diffusionsmesskopf zum Festellen von Gasen.**

(57) Bei einer Sonde zum Feststellen von Gasen ist eine Sintermetallplatte (15, 16) in einem Gehäuse (1) vorhanden, durch die die Gase Zutritt zu den Meßwendeln (6, 7) haben. Das Gehäuse umfaßt erfindungsgemäß einen die Meßwendel (6, 7) einschließenden Meßraum (4), dessen Wände (5) zum größeren Teil aus Sintermetall bestehen, und einen Anschlußraum (2), dessen Wände (3) aus massivem Metall bestehen und die Meßwendel (6, 7) tragen.

KRAFTWERK UNION AKTIENGESELLSCHAFT     Unser Zeichen

VPA 84 P 6 0 7 8 E

Sonde mit einem Diffusionsmeßkopf zum Feststellen von Gasen

Die Erfindung betrifft eine Sonde mit einem Diffusionsmeßkopf zum Feststellen von Gasen, die durch eine Sintermetallplatte Zutritt zu Meßwendeln in einem Gehäuse haben.

Eine solche Sonde ist in der DE-OS 30 46 560 beschrieben. Ihr Gehäuse, das die Meßwendel umschließt, besteht im wesentlichen aus einem würfelförmigen Körper aus Edelstahl, in dem eine zylindrische Ausnehmung eingearbeitet ist. Diese Ausnehmung ist an ihrem einen Ende mit einer Sintermetallplatte versehen, durch die die festzustellenden Gase Zutritt zu den dahinterliegenden Meßwendeln haben.

Die Erfindung geht von der Aufgabe aus, einen besseren Weg für die Gasbewegung im Bereich der Meßwendel zu schaffen. Dies kann zu einer Erhöhung der Meßgenauigkeit führen, weil Änderungen der Gaszusammensetzung sich schneller in dem Gehäuse bemerkbar machen. Allerdings ist dabei zu berücksichtigen, daß Sintermetalle nur schlecht zu bearbeiten sind.

Bei der erfindungsgemäßen Sonde ist vorgesehen, daß das Gehäuse einen die Meßwendel einschließenden Meßraum umfaßt, dessen Wände zum größeren Teil aus Sintermetall bestehen, und einen Anschlußraum, dessen Wände aus massivem Metall bestehen und die Anschlußleitungen der Sonde und die Meßwendel tragen.

Sm 2 Hgr / 18.10.1984

Bei der Erfindung ergibt sich durch die Trennung der beiden Räume eine Ausbildung, die zwar einen großen Durchlaßquerschnitt für die Gase ermöglicht, aber auch eine mechanisch feste und dennoch kostengünstige Herstellung gestattet, weil die Wände des Anschlußraumes für die erforderlichen mechanischen Verbindungen benutzt werden. Vorzugsweise sind die aus Sintermetall bestehenden Wände des Meßraumes mit Spanngliedern befestigt, die an den Wänden des Anschlußraumes angreifen.

Bei einer besonders günstigen Ausführungsform ist vorgesehen, daß die Wände des Meßraumes einen Zylinder aus Sintermetall umfassen, der an dem Anschlußraum abgestützt und mit einer Deckplatte an dem dem Anschlußraum abgekehrten Ende versehen ist. Dabei kann auch die Deckplatte aus Sintermetall bestehen. Dies bedeutet, daß der Meßraum bis auf die eine dem Anschlußraum zugekehrte Stirnseite des Zylinders allerseits gasdurchlässige Wände hat.

Der Meßraum kann vorteilhaft noch eine Temperaturmeßeinrichtung aufweisen. Mit ihr kann gegebenenfalls eine Zuordnung der Temperatur zu den Meßwerten des Diffusionsmeßkopfes erhalten werden, die für die Beurteilung der Gasmessung von Vorteil ist.

Eine andere Weiterbildung der Erfindung besteht darin, daß der Anschlußraum eine Öffnung aufweist, an deren Rand ein Rohrstück mit einem eingeklebten Kabel befestigt ist. Dabei kann man eine zusätzliche Abdichtung und/oder einen weiteren Befestigungseffekt dadurch erhalten, daß das Kabel einen über das Rohrstück greifenden Schrumpfschlauch trägt.

Zur näheren Erläuterung der Erfindung wird anhand der

0182064

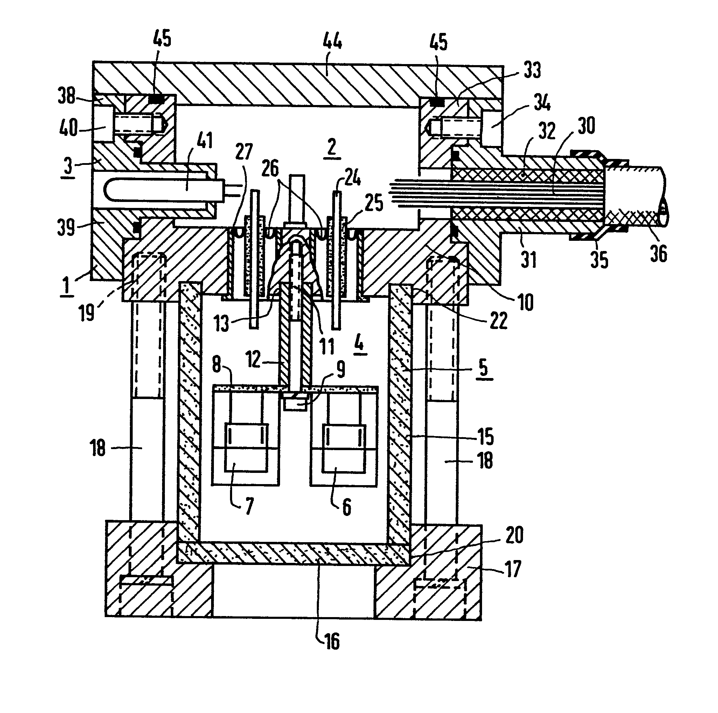

beiliegenden Zeichnung ein Ausführungsbeispiel beschrieben, das in der einzigen Figur in einem Schnitt
durch die Sonde dargestellt ist.

Die neue Sonde dient zur Feststellung von Wasserstoff
in einem Kernkraftwerk, der in feuchten $H_2$/Luft-Gemischen kontinuierlich und dauerhaft bis zu Konzentrationen von 10 Vol.% gemessen werden soll.

Die Sonde umfaßt ein Gehäuse 1, das aus zwei wesentlichen Teilen besteht. Der eine Teil bildet den Anschlußraum 2. Seine Wände 3 bestehen aus massivem Metall,
zum Beispiel aus V2A mit den aus der Figur ersichtlichen Wandstärken von einigen Millimetern. Der andere ist der Meßraum 4. Seine Wände 5 bestehen aus
Sintermetall.

Der Meßraum 4 umschließt Meßwendel 6 und 7, die an
einer Isolierstoffplatte 8 angebracht sind. Die Isolierstoffplatte 8 ist mit einer Schraube 9 an der dem
Meßraum 4 zugekehrten Wand 10 des Anschlußraumes 2 befestigt, die dort in eine Gewindebohrung 11 mit $M_4$-
Gewinde eingreift. Auf der Schraube 9 sitzt eine Buchse 12, die in eine Vertiefung 13 der Wand 10 eingreift
und damit Kräfte in seitlicher Richtung aufnimmt und
für den richtigen Abstand der Meßwendel 6, 7 von der
Wand 10 sorgt.

Die Wände 5 des Meßraumes 4 werden von einem Sintermetallzylinder 15 mit einem Durchmesser von etwa
60 mm gebildet, dessen der Wand 10 abgekehrte Stirnseite mit einer Sintermetallplatte 16 verschlossen
ist. Die Dicke der Sintermetallwände 15, 16 beträgt zum
Beispiel 5 mm.

Wie die Figur deutlich zeigt, wird die Sintermetall-

0182064

platte 16 mit einem massiven Metallring 17 angepreßt, der mit Schrauben 18 befestigt wird. Die Schrauben 18 greifen in Gewindebohrungen 19 der Wand 10.

Der Ring 17 hat eine Eindrehung 20, mit der die Platte 16 und der Zylinder 15 geführt werden. Eine weitere Eindrehung 22 in der Wand 10 bestimmt die Lage der Wände 5 des Meßraumes 4. Somit ist für die bearbeitungstechnisch schwierigen Sintermetallwände trotz ihrer Großflächigkeit eine stabile Bauweise sichergestellt.

Neben der Gewindebohrung 11 der Schraube 9 ist in der Wand 10 ein Durchbruch vorgesehen, in dem elektrische Durchführungsleiter 24 mit einer keramischen Isolierstoffschicht 25 über verschweißte Metallmembranen 26 in einem Isolierstoffkörper 27 sitzen, der in die Wand 10 eingesetzt und metallurgisch abgedichtet ist. Die Leiter 24 bilden die Verbindung zwischen den Meßwendeln 6 und 7 und dem Anschlußraum 2. Dort erfolgt der Anschluß eines Kabels 30, das in einem rohrförmigen Kabelstutzen 31 mit einer geeigneten Vergußmasse 32 dicht eingeklebt ist.

Der Kabelstutzen 31 sitzt in der Wand 33 des Anschlußraumes 2. Er besteht, wie die Wand 33, aus massivem Metall, zum Beispiel aus V2A, so daß dort eine sichere Befestigung mit Schrauben 34 vorgenommen werden kann. Über den Kabelstutzen 31 greift ein Schrumpfschlauch 35, der sich über den Mantel 36 des Kabels 30 erstreckt.

In der gegenüberliegenden Wand 38 des Anschlußraumes 2 ist ein Einsatz 39 mit Schrauben 40 befestigt. Dieser enthält einen Temperaturfühler 41, der wie die Meßwendel 6 und 7 mit den Leitern des Kabels 30 verbunden ist.

Die dem Meßraum 4 abgekehrte Seite des Anschlußraumes 2 ist mit einer Deckelplatte 44 verschlossen. Die zu ihrer Befestigung verwendeten Schrauben sind nicht dargestellt. Es ist jedoch ersichtlich, daß nach dem Abnehmen der Deckelplatte 44 der Anschlußraum 2 großflächig offengelegt ist, so daß dort die Verbindungen zwischen den Meßwendeln 6, 7, dem Temperaturfühler 41 und dem Kabel 30 ohne Schwierigkeiten zum Beispiel in Form einer dauerhaften Lötung günstig hergestellt werden können.

Die Meßwendel 6, 7 der neuen Sonde sind in dem großen Meßraum 4 großflächig mit der Umgebung verbunden, so daß die Änderungen der Gasatmosphäre, die festgestellt werden sollen, entsprechend schnell erfaßt werden können. Die bei Messungen entstehenden Reaktionsprodukte können gleichzeitig schnell abgeleitet werden, so daß die Messung nicht beeinträchtigt wird. Außerdem kann der die Meßwendel in bekannter Weise durchfließende Strom ohne unzulässige Erwärmung gegenüber bekannten Ausführungen gesteigert werden, so daß auch hierdurch die Meßgenauigkeit verbessert wird. Dabei kann der Temperatureinfluß der Umgebung durch den Temperaturfühler 41 unmittelbar berücksichtigt werden.

Der elektrische Anschlußraum 2 ist durch die Verwendung von störfallfesten Dichtungen 45 und durch die eingeschweißten Durchführungen 24, 25 gas- und feuchtigkeitsdicht abgeschlossen, so daß die für den jahrelangen Betrieb in einem Kernkraftwerk an unzugänglichen Stellen erforderliche Betriebssicherheit gegeben ist. Dabei sorgt der seitliche Anschluß des Kabels 32 nicht nur für eine einfache Herstellung der Anschlüsse

- 6 -     VPA 84 P 6 0 7 8 E

sondern auch durch die vergossene und mit dem Schrumpfschlauch 35 zusätzlich geschützte Ausführung für eine
absolute Dichtigkeit.

10 Patentansprüche
1 Figur

Patentansprüche

1. Sonde mit einem Diffusionsmeßkopf zum Feststellen von Gasen, die durch eine Sintermetallplatte Zutritt zu Meßwendeln in einem Gehäuse haben, d a d u r c h g e k e n n z e i c h n e t, daß das Gehäuse (1) einen die Meßwendel (6, 7) einschließenden Meßraum (4) umfaßt, dessen Wände (5) zum größeren Teil aus Sintermetall bestehen, und einen Anschlußraum (2), dessen Wände (3) aus massivem Metall bestehen und die Anschlußleitungen (30) der Sonde und die Meßwendel (6, 7) tragen.

2. Sonde nach Anspruch 1, d a d u r c h g e k e n n - z e i c h n e t, daß die aus Sintermetall bestehenden Wände (15, 16) des Meßraumes (4) mit Spanngliedern (18) befestigt sind, die an den Wänden (3) des Anschlußraumes (2) angreifen.

3. Sonde nach Anspruch 2, d a d u r c h g e k e n n - z e i c h n e t, daß die Wände (5) des Meßraumes (4) einen Zylinder (15) aus Sintermetall umfassen, der an dem Anschlußraum (2) abgestützt und mit einer Deckplatte (16) an dem dem Anschlußraum (2) abgekehrten Ende versehen ist.

4. Sonde nach Anspruch 3, d a d u r c h g e k e n n - z e i c h n e t, daß die Deckplatte (16) aus Sintermetall besteht.

5. Sonde nach den Ansprüchen 1 bis 4, d a d u r c h g e k e n n z e i c h n e t, daß der Anschlußraum (2) eine Temperaturmeßeinrichtung (41) aufweist.

0182064

6. Sonde nach den Ansprüchen 1 bis 5, d a d u r c h
g e k e n n z e i c h n e t, daß der Anschlußraum (2)
eine Öffnung aufweist, an deren Rand ein Rohrstück
(31) mit einem eingeklebten Kabel (30) befestigt ist.

7. Sonde nach Anspruch 6, d a d u r c h g e k e n n -
z e i c h n e t, daß das Rohrstück (31) an einer dem Meßraum (4) benachbarten Wand befestigt ist, und daß an
der dem Meßraum (4) abgekehrten Wand eine Öffnung mit
einer großflächigen Deckelplatte (44) verschlossen
ist.

8. Sonde nach Anspruch 6 oder 7, d a d u r c h g e -
k e n n z e i c h n e t, daß im Bereich der Öffnungen
Ringdichtungen (45) vorgesehen sind.

9. Sonde nach Anspruch 6, 7 oder 8, d a d u r c h g e -
k e n n z e i c h n e t, daß das Kabel (30) einen
über das Rohrstück (31) greifenden Schrumpfschlauch
(35) trägt.

10. Sonde nach einem der Ansprüche 1 bis 9, d a d u r c h
g e k e n n z e i c h n e t, daß die Meßwendel (6, 7)
über eingeschweißte keramische Durchführungen (24, 25)
mit dem Anschlußraum (2) verbunden sind.

## Europäisches Patentamt

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A,D | DE-A-3 046 560 (KRAFTWERK UNION AG)<br>* Ganzes Dokument * | 1-4 | G 01 N 27/16<br>G 21 D 3/04<br>G 01 N 33/00 |
| A,P | EP-A-0 133 502 (KRAFTWERK UNION AG)<br>* Zusammenfassung; Seite 1, Zeilen 7-10; Seite 2, Zeilen 20-23; Seite 3, Zeile 34 - Seite 4, Zeile 19; Seite 5, Zeilen 5-15; Ansprüche; Figur 1 * | 1,3-6 | |
| A | DE-A-2 745 034 (CHARBONNAGES DE FRANCE)<br>* Seite 6, Zeilen 1-9; Seite 16, Zeilen 1-7; Figuren 4-6 * | 1 | |
| A | GB-A-1 436 422 (ELECTRICAL REMOTE CONTROL CO. LIMITED)<br>* Seite 1, Zeilen 48-56; Seite 1, Zeile 81 - Seite 2, Zeile 2; Figuren 1, 2 * | 1 | RECHERCHIERTE SACHGEBIETE (Int. Cl.4)<br><br>G 01 N 27/00<br>G 21 D 3/00<br>G 01 M 3/00<br>G 01 N 33/00 |
| A | FR-A-2 353 846 (AUERGESELLSCHAFT G.M.B.H.)<br>* Seite 1, Zeilen 1-5; Seite 2, Zeilen 22-32; Ansprüche, Figur 1 * | 1 | |

Der vorliegende Recherchenbericht wurde für alle Patentansprüche erstellt.

| Recherchenort<br>BERLIN | Abschlußdatum der Recherche<br>24-01-1986 | Prüfer<br>VINSOME R M |
|---|---|---|